(19) Europäisches Patentamt / European Patent Office / Office européen des brevets

(11) **EP 4 458 336 A1**

(12) **EUROPEAN PATENT APPLICATION**
published in accordance with Art. 153(4) EPC

(43) Date of publication:
**06.11.2024 Bulletin 2024/45**

(21) Application number: **22915976.9**

(22) Date of filing: **23.12.2022**

(51) International Patent Classification (IPC):
**A61F 13/49** (2006.01)     **D01F 6/70** (2006.01)

(52) Cooperative Patent Classification (CPC):
**A61F 13/49; D01F 6/70**

(86) International application number:
**PCT/JP2022/047747**

(87) International publication number:
**WO 2023/127753 (06.07.2023 Gazette 2023/27)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **27.12.2021 JP 2021213222**

(71) Applicant: **Asahi Kasei Kabushiki Kaisha
Tokyo 1000006 (JP)**

(72) Inventor: **GOTO, Hideyuki
Tokyo 100-0006 (JP)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(54) **GATHER MEMBER AND HYGIENE PRODUCT INCLUDING SAME**

(57)     Provided are: a gather member that, when worn, does not easily leave a mark on the skin and tends to stay in place despite the body movement of the wearer; and a hygiene product. The present invention pertains to a gather member configured such that the ratio of the 50% elongation stress at 2000%/min elongation speed to the 50% elongation stress at 200%/min elongation speed is 1.05-3.00, and also pertains to a hygiene product including said gather member. Said gather member preferably includes at least thermoplastic polyurethane elastic fibers and a non-woven fabric.

EP 4 458 336 A1

**Description**

FIELD

The present invention relates to a gather member and a hygiene product including the same.

BACKGROUND

Conventionally, stretchable gather members have been arranged in the waist and legs of paper diapers to prevent shift when worn and to prevent leakage of urine or the like. Such gather members generally include a non-woven fabric and elastic fibers of, for example, rubber or polyurethane, and when producing such a gather member, the elastic fibers in an elongated state are bonded to the non-woven fabric using a hot-melt adhesive or the like, thereby imparting stretchability to the gather member. In general, gather members of hygiene products such as paper diapers are required to be not only stretchable but also unlikely to shift or leave marks.

For example, Patent Literature 1 below proposes a pull-up diaper which, when worn, is unlikely to shift and can be suppressed from leaving elastic marks since physical properties of a gather member having elastic fibers arranged at regular intervals between two sheets are specified.

[CITATION LIST]

[PATENT LITERATURE]

[PTL 1] Japanese Unexamined Patent Publication (Kokai) No. 2004-81365

SUMMARY

[TECHNICAL PROBLEM]

However, the pull-up diaper described in Patent Literature 1 cannot be sufficiently suppressed from shifting caused by strenuous movements of the wearer.

In light of the problems of the prior art described above, an object of the present invention is to provide a gather member and a hygiene product which are unlikely to leave marks when worn and which are unlikely to shift when the wearer moves.

[SOLUTION TO PROBLEM]

As a result of rigorous investigation in order to achieve this object, the present inventors have unexpectedly discovered that this object can be achieved by a gather member in which a ratio of a 50% elongation stress at a 2000%/min elongation speed to a 50% elongation stress at a 200%/min elongation speed is 1.05 or more and 3.00 or less, and have completed the present invention.

Specifically, the present invention is as described below.

[1] A gather member, wherein a ratio of a 50% elongation stress at a 2000%/min elongation speed to a 50% elongation stress at a 200%/min elongation speed is 1.05 or more and 3.00 or less.

[2] The gather member according to [1], comprising at least thermoplastic polyurethane elastic fibers and a non-woven fabric.

[3] The gather member according to [2], further comprising a hot-melt adhesive.

[4] The gather member according to [2] or [3], wherein a distance between hard domains of the thermoplastic polyurethane elastic fibers as measured with a small-angle X-ray scattering device is 10.0 nm or more and 30.0 nm or less, and a full width at half maximum of a peak of an azimuthal angle distribution is 50° or more and 130° or less.

[5] The gather member according to any one of [2] to [4], wherein a filament number of the thermoplastic polyurethane elastic fibers is 10 or more and 150 or less.

[6] The gather member according to any one of [2] to [5], wherein a single-strand fineness of the thermoplastic polyurethane elastic fibers is 5 dtex or more and 20 dtex or less.

[7] The gather member according to any one of [2] to [6], wherein a total fineness of the thermoplastic polyurethane elastic fibers is 150 dtex or more and 1500 dtex or less.

[8] The gather member according to any one of [2] to [7], wherein the thermoplastic polyurethane elastic fibers contain a polyurethane synthesized from polymer polyol, MDI, and 1,4-butanediol.

[9] The gather member according to any one of [2] to [8], wherein a molecular weight of hard segments of the thermoplastic polyurethane elastic fibers is 750 or more and 1500 or less.

[10] The gather member according to any one of [2] to [9], wherein the thermoplastic polyurethane elastic fibers have substantially no crosslinks containing an allophanate bond.

[11] The gather member according to any one of [3] to [10], wherein a content of the hot-melt adhesive is 0.02 g/m or more and 0.10 g/m or less relative to a length upon elongation per thermoplastic polyurethane elastic fiber.

[12] The gather member according to any one of [3] to [11], wherein the hot-melt adhesive is a styrene block copolymer selected from the group consisting of block copolymers of a vinyl aromatic hydrocarbon and a conjugated diene compound and hydrogenated products thereof.

[13] The gather member according to any one of [2] to [12], wherein a basis weight of the non-woven fabric is 8 $g/m^2$ or more and 50 $g/m^2$ or less.

[14] The gather member according to any one of [1] to [13], wherein a full elongation rate of the gather member is 50% or more and 250% or less.

[15] A hygiene product comprising the gather member according to any one of [1] to [14].

[ADVANTAGEOUS EFFECTS OF INVENTION]

The gather member and hygiene product of the present invention are unlikely to leave marks when worn and are unlikely to shift when the wearer moves.

DESCRIPTION OF EMBODIMENTS

The embodiment for carrying out the present invention (hereinafter referred to as "the present embodiment") will be described in detail below. The present invention is not limited to the present embodiment below, and various changes can be made within the scope of the spirit thereof.

In the gather member of the present embodiment, a ratio of a 50% elongation stress at a 2000%/min elongation speed to a 50% elongation stress at a 200%/min elongation speed is 1.05 or more and 3.00 or less.

[Gather Member]

In the gather member of the present embodiment, the ratio of the 50% elongation stress at an elongation speed of 2000%/min to the 50% elongation stress at an elongation speed of 200%/min (hereinafter also referred to as the "speed response ratio") is 1.05 or more and 3.00 or less, more preferably 1.06 or more and 2.90 or less, further preferably 1.07 or more and 2.80 or less, further preferably 1.10 or more and 2.50 or less, further preferably 1.15 or more and 2.20 or less, and most preferably 1.20 or more and 2.0 or less.

In the gather member of the present embodiment, since the speed response ratio is set to 1.05 or more, the stress increases specifically when the wearer moves and the gather member is elongated at high speed, whereby the gather member exhibits unlikeliness to shift when the wearer moves. Furthermore, since the speed response ratio is set to 3.00 or less, excessive stress is not exerted on the gather member when elongated at high speed, and is unlikely to leave marks when worn.

The gather member of the present embodiment is a stretchable member having a large number of folds, and stretches and shrinks in at least one direction. The materials constituting the gather member and the method for producing the gather member are not particularly limited, and the gather member can be produced using methods which are conventionally known in the production of ordinary paper diapers, sanitary products, and the like. Generally, the gather member can be produced by a method in which elastic fibers in an elongated state are bonded to a non-woven fabric or the like using hot melt or the like. As a specific example of the method for producing the gather member, there is a method including the steps of unwinding elastic fibers from a spool, drawing the elastic fibers through guides or rollers, directly applying a hot-melt adhesive to the elastic fibers in a drawn state, bonding the elastic fibers to a non-woven fabric by applying pressure with a pressure roller, and shrinking an elastic member.

In the gather member of the present embodiment, from the viewpoint of improving stretch recovery properties, it is preferable that the thermoplastic polyurethane elastic fibers and the non-woven fabric be bonded together. Examples of methods for bonding the thermoplastic polyurethane elastic fibers and the non-woven fabric include a method of using a hot-melt adhesive and a method of using heat to melt and bond some or all of the thermoplastic polyurethane elastic fibers and/or the non-woven fabric. Among these, in the gather member of the present embodiment, from the viewpoint of improving stretch recovery properties, it is preferable that the thermoplastic polyurethane elastic fibers and the non-woven fabric be bonded with a hot-melt adhesive. The thermoplastic polyurethane elastic fibers and the non-woven fabric may be bonded continuously over the entire lengths thereof in the longitudinal direction of the thermoplastic polyurethane elastic fibers, or may be bonded intermittently and partially in the longitudinal direction of the polyurethane elastic fibers. Furthermore, a material other than the non-woven fabric and the polyurethane elastic fibers may be inserted, bonded, or applied within the range that does not impede the object of the present invention.

In the gather member of the present embodiment, when a plurality of thermoplastic polyurethane elastic fibers are bonded to the non-woven fabric, the polyurethane elastic fibers may be bonded to the non-woven fabric in parallel to each other, or may be arranged in arcs, parabolas, zigzags, or sinusoids, or a combination of these.

The full elongation rate of the gather member of the present embodiment is preferably 50% or more and 250% or less from the viewpoint of increasing the speed response ratio. Since the full elongation rate of the gather member is set to 50% or more, tension in the non-woven fabric can be prevented and the speed response ratio of the thermoplastic polyurethane elastic fibers is easily reflected in the gather member, whereby the speed response ratio of the gather member can be increased. Since the full elongation rate of the gather member is set to 250% or less, the folds of the gather member can be reduced and the speed response ratio of the thermoplastic polyurethane elastic fibers when the gather member is elongated is easily reflected in the gather member, whereby the speed response ratio of the gather member can be increased. The method for setting the full elongation rate of the gather member to 50% or more and 250% or less is not particularly limited, and for example, a method for producing a gather member via controlling the draw ratio of the thermoplastic polyurethane elastic fibers before bonding to the non-woven fabric is preferably used.

[Thermoplastic Polyurethane Elastic Fibers Contained in Gather Member]

The gather member of the present embodiment can contain thermoplastic polyurethane elastic fibers. The thermoplastic polyurethane elastic fibers preferably contain a thermoplastic polyurethane in an amount of 80 wt% or more, more preferably 85 wt% or more, further preferably 90 wt%, and most preferably 95 wt% or more. The thermoplastic polyurethane need only have thermoplasticity, and is not particularly limited as long as it has, for example, a structure polymerized from a diisocyanate, a polymer polyol, a low molecular weight diol, and a low molecular weight diamine. Furthermore, the polymerization method is also not particularly limited. The thermoplastic polyurethane may be, for example, a polyurethane (hereinafter also referred to as "polyurethane urea") polymerized from a diisocyanate, a polymer polyol, and a low molecular weight diamine as a chain extender consisting of an active hydrogen compound. Alternatively, it may be a polyurethane (hereinafter also referred to as "polyurethane urethane") polymerized from a diisocyanate, a polymer polyol, and a low molecular weight diol as a chain extender consisting of an active hydrogen compound. Trifunctional or higher functional glycols and isocyanates may be used as long as they do not interfere with the desired effects of the present invention. As used herein, "thermoplastic" means reversible properties, in which it can be melted by heating below the decomposition temperature, exhibits plastic flow while in a molten state, and solidifies upon cooling. Generally, polyurethane resins begin to decompose at temperatures of 230 °C or higher.

Examples of polymer polyols include, but are not limited to, polymer diols such as polyether diols, polyester diols, and polycarbonate diols. From the viewpoint of hydrolysis resistance, the polymer polyol is preferably a polyether polyol, and more preferably a polyether diol.

Examples of polyether polyols include polyethylene oxide, polyethylene glycol, polyethylene glycol derivatives, polypropylene glycol, polytetramethylene ether glycol, copolymerized diols consisting of tetrahydrofuran (THF) and neopentyl glycol, and copolymer diols consisting of THF and 3-methyltetrahydrofuran. These polyether polyols may be used alone or in combination of two or more thereof. Furthermore, the number average molecular weight of the polymer diol is preferably 1000 or more and 8000 or less. By using a polymer diol within this range, elastic fibers with excellent elongation, stretch recovery properties, and heat resistance can easily be obtained. From the viewpoint of light embrittlement, the polyether polyol is preferably polytetramethylene ether glycol, a copolymerized diol which is a copolymer of THF and neopentyl glycol, or a polyol which is a blend of these.

Examples of diisocyanates include aromatic diisocyanates, alicyclic diisocyanates, and aliphatic diisocyanates. Examples of the aromatic diisocyanate include, but are not limited to, diphenylmethane diisocyanate (hereinafter also referred to as "MDI"), tolylene diisocyanate, 1,4-diisocyanate benzene, xylylene diisocyanate, and 2,6-naphthalene diisocyanate. Examples of alicyclic diisocyanates and aliphatic diisocyanates include methylene bis(cyclohexyl isocyanate) (hereinafter also referred to as "H12MDI"), isophorone diisocyanate, methylcyclohexane 2,4-diisocyanate, methylcyclohexane 2,6-diisocyanate, cyclohexane 1,4-diisocyanate, hexahydroxylylene diisocyanate, hexahydrotolylene diisocyanate, and octahydro 1,5-naphthalene diisocyanate. These diisocyanates may be used alone or in combination of two or more thereof. In particular, from the viewpoint of the stretch recovery properties of elastic fibers, the diisocyanate is preferably an aromatic diisocyanate, and more preferably MDI.

The chain extender consisting of an active hydrogen compound is preferably at least one selected from the group consisting of low molecular weight diamines and low molecular weight diols. The chain extender may also have both a hydroxyl group and an amino group in its molecule, such as ethanolamine. From the viewpoint of obtaining a thermoplastic polyurethane suitable for melt-spinning, the active hydrogen compound is preferably a low molecular weight diol.

Examples of the low molecular weight diamine as a chain extender consisting of an active hydrogen compound include, but are not limited to, hydrazine, ethylenediamine, 1,2-propanediamine, 1,3-propanediamine, 2-methyl-1,5-pentanediamine, 1,2-diaminobutane, 1,3-diaminobutane, 1-amino-3,3,5-trimethyl-5-aminomethylcyclohexane, 2,2-dimethyl-1,3-diaminopropane, 1,3-diamino-2,2-dimethylbutane, 2,4-diamino-1-methylcyclohexane, 1,3-pentanediamine, 1,3-cyclohexanediamine, bis(4-aminophenyl)phosphine oxide, hexamethylenediamine, 1,3-cyclohexyldiamine, hexahydrometaphenylenediamine, 2-methylpentamethylenediamine, and bis(4-aminophenyl)phosphine oxide.

Examples of the low molecular weight diol as a chain extender consisting of an active hydrogen compound include ethylene glycol, 1,3-propanediol, 1,4-butanediol, bishydroxyethoxybenzene, bishydroxyethylene terephthalate, 1-methyl-1,2-ethanediol, and 1,6-hexanediol. These low molecular weight diols may be used alone or in combination of two or more thereof. From the viewpoint of the stretch recovery properties of elastic fibers, the low molecular weight diol is preferably a diol having a molecular weight of 60 or more and 120 or less, and more preferably 1,4-butanediol.

The thermoplastic polyurethane can be produced using a known polyurethanization reaction technique, and may be produced by either a one-shot method or a prepolymer method. In the case of the prepolymer method, the polymer polyol and diisocyanate are added to a reaction tank having a hot water jacket and a stirrer under a nitrogen purge at a molar ratio of preferably 1.0: 1.8 to 3.0, and more preferably 1.0:2.0 to 2.5, and the prepolymer reaction is preferably carried out at a temperature of 40 °C or higher and 100 °C or lower, and more preferably 50 °C or higher and 80 °C or lower, to obtain a prepolymer with isocyanate groups at both terminals thereof. Next, an active hydrogen compound is added to this prepolymer with isocyanate groups at both terminals thereof at an equivalent amount approximately equal to the number of functional groups of the isocyanate end groups to perform a chain extension reaction. The equivalent ratio is preferably 0.95 or more and 1.1 or less, and more preferably 0.99 or more and 1.05 or less, relative to the isocyanate terminal groups. Thereafter, solid phase polymerization is performed to obtain polyurethane of a predetermined molecular weight. As the methods for the chain extension reaction and solid phase polymerization that may be adopted, the active hydrogen compound is added as-is to a batch reaction vessel containing the prepolymer at preferably 40 °C or higher and 100 °C or lower, and the polymer is then discharged and subjected to solid phase polymerization preferably at a temperature of 60 °C or higher and 200 °C or lower, and more preferably 70 °C or higher and 150 °C or lower, and then, a chip-shaped polymer may be obtained by palletization. Alternatively, after uniformly mixing the prepolymer and the active hydrogen compound, using a cylindrical pipe configuration or a twin-screw extruder, a cylinder temperature in the polymerization zone is set to preferably 160 °C or higher and 240 °C or lower, the polymer is continuously or semi-continuously obtained, and thereafter, solid phase polymerization may be carried out preferably at a temperature of 60 °C or higher and 220 °C or lower, and more preferably 70 °C or higher and 150 °C or lower.

The spinning method for producing the thermoplastic polyurethane elastic fibers is not particularly limited as long as the desired physical properties can be obtained. In addition to a method of introducing the polyurethane elastic chips into an extruder, heating, and melt-spinning, there are other methods, for example, a method of melting the polyurethane elastic chips and then mixing a polyisocyanate compound and spinning, and a method for continuous spinning without chip formation, in which a reaction product of a prepolymer with isocyanate groups at both terminals thereof and an active hydrogen compound is added to a prepolymer with isocyanate groups at both terminals thereof.

The thermoplastic polyurethane introduced into the extruder is metered by a metering pump and introduced into a spinning head. If necessary, foreign substances are removed by filtration using a wire mesh or glass beads in the spinning head. Thereafter, discharging from the spinneret, air-cooling in a cold air chamber, and addition of a treatment agent if necessary, are followed by winding via a godet roll.

In the spinning step, the die temperature, cold air speed, cold air temperature, bundling position, and spinning speed are adjusted to precisely control the fiber temperature profile and spinning tension. The temperature of the die is preferably 180 °C to 220 °C, and more preferably 200 °C to 210 °C. A common melt-spinning cooling method is used, such as applying cold air from directly below the spinning nozzle perpendicularly to the strand running direction, the cold air speed is preferably 0.2 m/s to 2.0 m/s, and more preferably 0.5 m/s to 1.2 m/s, and the cold air temperature is preferably 5 °C to 20 °C, and more preferably 7 °C to 15 °C. Examples of the method for bundling the multifilaments include a method in which a false twister is installed between the spinneret and the godet roll, the twist is propagated from the bottom by varying the strength of the twist, the filaments are bundled together, and the height of the bundling point is controlled. The false twisting method can be selected from general methods. Air false twisting using an air nozzle or a ring false twisting machine involving contact with a rotating ring can be used.

A treatment agent such as an oil agent may be applied to the thermoplastic polyurethane elastic fibers from the viewpoint of the unwinding properties and processability. Examples of the treatment agent include, but are not limited to, silicone oils such as dimethyl silicone, mineral oils, and combinations thereof. The method of applying the treatment agent is not particularly limited, and examples thereof include a method of applying the treatment agent using an oiling roller.

The weight average molecular weight (Mw) of thermoplastic polyurethane elastic fibers, when measured by GPC against a polystyrene standard, is preferably 80,000 or more and 800,000 or less, more preferably 80,000 or more and 500,000 or less, and further preferably 100,000 or more and 300,000 or less.

The number average molecular weight (Mn) of thermoplastic polyurethane elastic fibers, when measured by GPC against a polystyrene standard, is preferably 40,000 or more and 400,000 or less, more preferably 40,000 or more and 250,000 or less, and further preferably 50,000 or more and 150,000 or less.

The polydispersity (Mw/Mn) of thermoplastic polyurethane elastic fibers, when measured by GPC against a polystyrene standard, is preferably 1.5 or more and 3.0 or less, more preferably 1.5 or more and 2.5 or less.

The thermoplastic polyurethane elastic fibers may contain a polymer other than polyurethane and additives such as antioxidants, light stabilizers, ultraviolet absorbers, gas discoloration inhibitors, dyes, activators, matting agents, and lubricants, as long as the desired effects of the present invention are not impaired thereby.

In the thermoplastic polyurethane elastic fibers, from the viewpoint of exhibiting the speed response ratio of the gather member, the distance between hard domains as measured with a small-angle X-ray scattering device (hereinafter referred to as "SAXS") is preferably 10.0 nm or more and 30.0 nm or less, more preferably 10.0 nm or more and 27.0 nm or less, further preferably 10.0 nm or more and 25.0 nm or less, further preferably 10.5 nm or more and 20.0 nm or less, and most preferably 11.0 nm or more and 15.0 nm or less, and the full width at half maximum of the peak of the azimuthal angle distribution is preferably 50° or more and 130° or less, more preferably 60° or more and 120° or less, further preferably 60° or more and 110° or less, and most preferably 80° or more and 100° or less. Since the distance between hard domains is set to 10.0 nm or more and 30.0 nm or less, it becomes easier to produce a gather member having a speed response ratio in a suitable range, whereby both the gather member is unlikely to shift when the wearer moves and marks are suppressed when worn. Though the reason why the distance between hard domains and the speed response ratio are correlated is not yet clear, the inventors presume as follows. It is considered that the speed response ratio increases with an increase in difference between the structural changes of the hard domains of the thermoplastic polyurethane elastic fibers when the gather member is elongated at high speed and the structural changes of the hard domains of the thermoplastic polyurethane elastic fibers when the gather member is elongated at low speed. When a gather member containing thermoplastic polyurethane elastic fibers having a long distance between hard domains and a large hard domain size is elongated at high speed, the hard domain structure of the polyurethane elastic fiber is significantly destroyed as compared to when the polyurethane elastic fiber is elongated at low speed, and the destruction stress improves the physical properties and increases the speed response ratio. Since the full width at half maximum is set to 50° or more and 130° or less, it becomes easier to produce a gather member having a speed response ratio of 1.05 or more, whereby unlikeliness of shift of the gather member can be exhibited when the wearer moves. Though the reason why it becomes easier to produce a gather member having a speed response ratio of 1.05 or more by setting the full width at half maximum to 50° or more and 130° or less is not yet clear, the inventors presume as follows. When a gather member containing thermoplastic polyurethane elastic fibers having a degree of orientation such that the full width at half maximum is 50° or more is elongated at high speed, the hard domain structure of the polyurethane elastic fibers is destroyed to a greater extent as compared to the case in which the polyurethane elastic fibers are elongated at low speed, and the destruction stress improves the physical properties and increases the speed response ratio. In a gather member containing thermoplastic polyurethane elastic fibers having a degree of orientation such that the full width at half maximum is 130° or less, there is little change in the structure of the hard domain during elongation at low speed, and the difference between the change in structures when elongated at relatively high speed is large, and the speed response ratio becomes large.

The method for setting the distance between hard domains of the thermoplastic polyurethane elastic fibers to 10.0 nm or more and 30.0 nm or less, and the full width at half maximum of the peak of the azimuthal angle distribution to 50° or more and 130° or less is not particularly limited, and for example, in the production of a gather member, a method in which the thermal history imparted to the thermoplastic polyurethane elastic fibers is controlled in a step of bonding the thermoplastic polyurethane elastic fibers and the non-woven fabric using a hot-melt adhesive or a step subsequent to the bonding is preferably used. Specifically, since the distance between hard domains tends to increase due to heat reception, a method using heated rollers in the step of bonding the thermoplastic polyurethane elastic fibers and the non-woven fabric using a hot-melt adhesive can be adopted. Furthermore, since the full width at half maximum of the peak of the azimuthal angle distribution tends to increase as the cooling rate increases after heat reception, a method of blowing cold air onto the gather member immediately after bonding the thermoplastic polyurethane elastic fibers and the non-woven fabric using a hot-melt adhesive can be adopted.

The filament number of the thermoplastic polyurethane elastic fibers is preferably 10 or more and 150 or less, more preferably 10 or more and 120 or less, and more preferably 20 or more and 120 or less, from the viewpoint of exhibiting the speed response ratio of the gather member. Though the reason why the speed response ratio can be increased by setting the filament number to 10 or more and 150 or less is not yet clear, the inventors presume as follows. Since thermoplastic polyurethane elastic fibers produced with a filament number of 10 or more have high fiber physical properties of raw yarns, the speed response ratio of the raw yarns can be easily reflected in the gather member. In thermoplastic polyurethane elastic fibers produced with a filament number of 150 or less, since the single strands can uniformly be cooled during the cooling process in the melt spinning step, variations in physical properties between single strands can be reduced, the speed response ratio of the raw yarns is easily increased, and the speed response ratio of the raw yarns is easily reflected in the gather member.

The single-strand fineness of the thermoplastic polyurethane elastic fibers is preferably 5 dtex or more and 20 dtex or less, and more preferably 7 dtex or more and 16 dtex or less, from the viewpoint of exhibiting the speed response ratio of the gather member. Though the reason why the speed response ratio can be increased by setting the single-strand fineness to 5 dtex or more and 20 dtex or less is not yet clear, the inventors presume as follows. In thermoplastic polyurethane elastic fibers produced with a single-strand fineness of 5 dtex or more, since the single strands can uniformly be cooled during the cooling process in the melt spinning step, variations in physical properties between single strands can be reduced, the speed response ratio of the raw yarns is easily increased, and the speed response ratio of the raw yarns is easily reflected in the gather member. Since thermoplastic polyurethane elastic fibers produced with a single-strand fineness of 20 dtex or less can easily be cooled during the cooling process in the melt spinning step, variations in physical property between single strands can be reduced, the speed response ratio of the raw yarns is easily increased, and the speed response ratio of the raw yarns is easily reflected in the gather member.

The total fineness of the thermoplastic polyurethane elastic fibers is preferably 150 dtex or more and 1500 dtex or less from the viewpoint of exhibiting the speed response ratio of the gather member. Though the reason why the speed response ratio can be increased by setting the total fineness of the thermoplastic polyurethane elastic fibers to 150 dtex or more and 1500 dtex or less is not yet clear, the inventors presume as follows. In thermoplastic polyurethane elastic fibers produced with a total fineness of 150 dtex or more, since the raw yarns have high physical properties, the speed response ratio of the raw yarns can easily be reflected in the gather member. Since thermoplastic polyurethane elastic fibers produced with a total fineness of 1500 dtex or less can be uniformly cooled during the cooling process in the melt spinning step, variations in physical properties in the strand length direction can be reduced, the speed response ratio of the raw yarns is easily increased, and the speed response ratio of the raw yarns is easily reflected in the gather member.

The thermoplastic polyurethane elastic fibers preferably contain a polyurethane synthesized from a polymer polyol, MDI, and 1,4-butanediol, from the viewpoint of increasing the speed response ratio of the gather member. Though the reason why the speed response ratio can be exhibited by including a polyurethane synthesized from a polymer polyol, MDI, and 1,4-butanediol in the thermoplastic polyurethane elastic fibers contained in the gather member of the present embodiment is not yet clear, the inventors presume as follows. It is considered that the speed response ratio increases with an increase in difference between structural changes in the hard domains of the thermoplastic polyurethane elastic fibers when the gather member is elongated at high speed and structural changes of the hard domains of the thermoplastic polyurethane elastic fibers when the gather member is elongated at low speed. The thermoplastic polyurethane elastic fibers synthesized from a polymer polyol, MDI, and 1,4-butanediol have strong hydrogen bonding strength in the hard domains, and the structure of the hard domains is greatly destroyed when elongated at high speed, whereas the hard domain structure is unlikely to be destroyed when elongated at low speed, whereby the speed response ratio can be increased.

The molecular weight of the hard segments of the thermoplastic polyurethane elastic fibers is, from the viewpoint of exhibiting the speed response ratio of the gather member, preferably 750 or more and 1500 or less, more preferably 800 or more and 1300 or less, and further preferably 850 or more and 1200 or less. Since the molecular weight of the hard segments of the thermoplastic polyurethane elastic fibers contained in the gather member of the present embodiment is set to 750 or more and 1500 or less, the speed response ratio can easily be set to 1.05 or more and 3.00 or less. Though the reason why the speed response ratio can be easily set to 1.05 or more and 3.00 or less by setting the molecular weight of the hard segments to 750 or more and 1500 or less is not yet clear, the inventors presume as follows. Since the molecular weight of the hard segments is set to 750 or more and 1500 or less, when the gather member containing the thermoplastic polyurethane elastic fibers is elongated at high speed, the degree of structural destruction of the hard domains of the polyurethane elastic fiber becomes appropriate, whereby the speed response ratio is controlled within a range of 1.05 or more and 3.00 or less. The method for producing thermoplastic polyurethane elastic fibers in which the molecular weight of the hard segments is 750 or more and 1500 or less is not particularly limited, and a method for producing thermoplastic polyurethane elastic fibers from thermoplastic polyurethane polymerized via controlling the number of moles of isocyanate relative to the polymer polyol is preferably used. Generally, by increasing the number of moles of isocyanate relative to the polymer polyol, the molecular weight of the hard segments can also be increased.

The thermoplastic polyurethane elastic fibers preferably have substantially no crosslinks containing an allophanate bond, in which a urethane bond is crosslinked with an isocyanate group, from the viewpoint of further enhancing the effect of improving unlikeliness of shift of the gather member when the wearer moves. Though the reason why the speed response ratio can be increased by having no crosslinks containing an allophanate bond is not yet clear, the inventors presume as follows. It is considered that the speed response ratio increases with an increase in difference between the structural changes of the hard domains of the thermoplastic polyurethane elastic fibers when the gather member is elongated at high speed and the structural changes of the hard domains of thermoplastic polyurethane elastic fibers when the gather member is elongated at low speed. When a gather member containing thermoplastic polyurethane elastic fibers which contain no crosslinked structure is elongated at high speed, the hard domain structure is easily destroyed when elongated at high speed, whereby the speed response ratio becomes high, and unlikeliness of shift of the gather member is exhibited when the wearer moves. The method for producing thermoplastic polyurethane elastic fibers having substantially no crosslinks containing an allophanate bond is not particularly limited, and for example, a method for producing thermoplastic polyurethane elastic fibers from a thermoplastic polyurethane polymerized via controlling the total number of moles of a polymer polyol and a chain extender consisting of an active hydrogen compound to be equal to or greater than the number of moles of isocyanate is preferably used.

[Hot-Melt Adhesive Contained in Gather Member]

The amount of hot-melt adhesive contained in the gather member of the present embodiment is, from the viewpoint of increasing the speed response ratio of the gather member, preferably 0.02 g/m or more and 0.10 g/m or less, and more preferably 0.03 g/m or more and 0.08 g/m or less relative to a length upon elongation per thermoplastic polyurethane elastic fiber.

The hot-melt adhesive is preferably a hot-melt adhesive containing a thermoplastic elastomer as a main component, and specifically, containing preferably 60 wt% or more, more preferably 65 wt% or more, further preferably 70 wt% or more, and most preferably 75 wt% or more of a thermoplastic elastomer. Examples of hot-melt adhesives containing a thermoplastic elastomer as a main component include rubber-based hot-melt adhesives in which a styrene-based block copolymer composed of a block copolymer of a vinyl aromatic hydrocarbon and a conjugated diene compound and hydrogenated products thereof is used, rubber-based hot-melt adhesives in which a polyolefin copolymer is used, and hot-melt adhesives in which a copolymer containing ethylene as the main chain (ethylene-vinyl acetate copolymers (EVA), ethylene-ethyl acrylate copolymers (EEA), and ethylene-n-butyl copolymers (EnBA)) is used. Among these, from the viewpoint of increasing the speed response ratio of the gather member, the hot-melt adhesive contained in the gather member of the present embodiment is preferably a styrene-based block copolymer composed of a block copolymer of a vinyl aromatic hydrocarbon and a conjugated diene compound and hydrogenated products thereof. Though the reason why the speed response ratio can be increased by using a styrene-based block copolymer composed of a block copolymer of a vinyl aromatic hydrocarbon and a conjugated diene compound and hydrogenated products thereof is used as the hot-melt adhesive contained in the gather member is not yet clear, the inventors presume as follows. Since the intermolecular force acting on the aromatic hydrocarbon of the hot-melt adhesive and the aromatic part contained in the hard domains of thermoplastic polyurethane elastic fibers becomes stronger, the adhesive force between thermoplastic polyurethane elastic fibers and the non-woven fabric becomes stronger, and thus, the speed response ratio of the yarns is easily reflected in the gather member.

Specific examples of a styrene-based block copolymers composed of a block copolymer of a vinyl aromatic hydrocarbon and a conjugated diene compound and hydrogenated products thereof include unhydrogenated additives of block copolymers such as styrene-isoprene-styrene block copolymers (SIS) and styrene-butadiene-styrene block copolymers (SBS); and hydrogen additives such as styrene-butadiene/butylene-styrene block copolymers (SBBS), styrene-ethylene/butylene-styrene block copolymers (SEBS), styrene-ethylene/propylene-styrene block copolymers (SEPS), and styrene-ethylene-ethylene/propylene-styrene block copolymers (SEEPS). These styrene-based block copolymers may be used alone or in combination of two or more thereof.

The hot-melt adhesive may contain additives such as antioxidants, light stabilizers, ultraviolet absorbers, lubricants, plasticizers, and lubricants, as long as the desired effects of the present invention are not impaired thereby.

In the production of the gather member of the present embodiment, the bonding method using the hot-melt adhesive is not particularly limited, and conventionally known methods can be adopted. Generally, hot-melt adhesives are heated and melted in a heated melting tank before coating various adherends. Examples of the method for the coating with the hot-melt adhesive include methods such as applying with a comb gun such as V-slit, SureWrap, bead, summit, spiral, and pattern coating.

The melt viscosity of the hot-melt adhesive is not particularly limited, and is preferably 1,000 mPa·s or more and 30,000 mPa·s or less at 150 °C. When the melt viscosity of the hot-melt adhesive at 150 °C is 1,000 mPa·s or more, the adhesiveness due to the hot-melt adhesive is improved, whereby the polyurethane elastic fibers do not peel off from the non-woven fabric. When the melt viscosity of the hot-melt adhesive at 150 °C is 30,000 mPa·s or less, the texture of the gather member is improved.

[Non-Woven Fabric Contained in Gather Member]

The type of the non-woven fabric used in the gather member of the present embodiment is not particularly limited, the non-woven fabric may be a long-fiber non-woven fabric such as a spunbond non-woven fabric, a melt-blown non-woven fabric, a flash-spun non-woven fabric, an electrospun non-woven fabric, or a wet-spun non-woven fabric, and may be a short-fiber non-woven fabric obtained by a wet papermaking method, but a long-fiber non-woven fabric is preferable, and a spunbond non-woven fabric is particularly preferable. The basis weight of the non-woven fabric is not limited, and is preferably 8 g/m$^2$ or more and 50 g/m$^2$ or less from the viewpoint of increasing the speed response ratio of the gather member. The reason why the speed response ratio can be increased by setting the basis weight of the non-woven fabric contained in the gather member to 8 g/m$^2$ or more and 50 g/m$^2$ or less is not yet clear, the inventors presume as follows. Since the basis weight of the non-woven fabric is set to 8 g/m$^2$ or more, when bonding thermoplastic polyurethane elastic fibers and the non-woven fabric with a hot-melt adhesive, seeping of the hot-melt adhesive into the non-woven fabric can be suppressed, the adhesive strength between the hot-melt adhesive and thermoplastic polyurethane elastic fibers can be increased, and thus, the speed response ratio of the yarns can easily be reflected in the gather member. Furthermore, since the basis weight of the non-woven fabric is set to 50 g/m$^2$ or less, a soft gather member can be obtained and the gather member is easily elongated following the yarns, whereby the speed response ratio of the yarns is easily reflected in the gather member.

The fibers constituting the non-woven fabric are not particularly limited, and they may be polyolefin fibers such as polyethylene or polypropylene fibers, polyester fibers, polyamide fibers, cellulose fibers, or polyurethane fibers, a mixture of these may be used, and polyolefin fibers are preferable. A combination of two or more types of non-woven fabrics composed of different fibers may be used. As the polyolefin fibers, for example, fibers composed of a resin such as a polyethylene or polypropylene resin, or a copolymer of a monomer thereof and another $\alpha$-olefin can be used. Among these, it is preferable to use polypropylene fibers composed of a polypropylene and/or a polypropylene copolymer because they are strong, unlikely to break, and have excellent dimensional stability when producing stretchable materials.

The non-woven fabric preferably has high flexibility. In order to impart high flexibility to the non-woven fabric, it is preferable to use partial thermocompression bonding as the bonding method when bonding the fiber webs to form the non-woven fabrics, and the smaller the area ratio of the partial thermocompression bonding, the better. Furthermore, the average single-strand fineness of the fibers constituting the non-woven fabric is preferably 0.5 dtex or more and 3.0 dtex or less. In order to obtain suitable flexibility, the non-woven fabric may be textured by a shaping process. Regarding the shape in the shaping process, straight lines, curves, corners, circles, satin-like shapes, and other continuous and discontinuous shapes can be applied, and from the viewpoint of flexibility effect, a tortoiseshell pattern is preferable. Furthermore, in order to obtain suitable flexibility, an ester compound of a trivalent to hexavalent polyol and a monocarboxylic acid may be contained in the non-woven fabric. The ester compound may be mixed with and contained in the raw material resin before spinning, or may be added and contained after producing the non-woven fabric.

The non-woven fabric may contain a hydrophilizing agent. Furthermore, the fibers constituting the non-woven fabric may contain nucleating agents, flame retardants, inorganic fillers, pigments, colorants, heat stabilizers, and antistatic agents as long as they do not impede the object of the present invention.

[Hygiene product]

One aspect of the present invention provides a hygiene product comprising the gather member of the present embodiment. Specific examples of the hygiene product include absorbent articles represented by disposable paper diapers and sanitary products. In paper diapers, the gather member of the present embodiment can suitably be used in the waist region and the leg region.

EXAMPLES

The present invention will be specifically described using the following Examples and Comparative Examples, but the scope of the present invention is not limited by the Examples.

First, the evaluation methods used in the following Examples will be described.

(1) Speed Response Ratio of Gather Member

Using a tensile tester (EZ-SX AUTOGRAPH manufactured by Shimadzu Corporation) at 20 °C and 65% RH, a gather member is cut to a size of 2 cm in width, set in a tensile testing machine so that the initial length is 5 cm without deflection and the initial load is 0 cN, elongated to 100% elongation at elongation speeds of 200%/min and 2000%/min, and the stress at 50% elongation (cN) is measured. The maximum stress at an elongation speed of 200%/min is defined as R, the maximum stress at an elongation speed of 2000%/min is defined as R', and speed response ratio of the gather member is calculated from the following calculation formula:

$$\text{Speed response ratio} = R'/R$$

The tensile tests at elongation speeds of 200%/min and 2000%/min are performed using the respective different samples.

(2) Hot-Melt Adhesive Content of Gather Member

A sample is cut out from a gather member so as to contain one thermoplastic polyurethane elastic fiber and have a length of 10 cm, and stored in an environment of 20 °C and 65% RH for 24 hours, and then, the weight X (g) thereof is measured. The sample is immersed in 100 mL of cyclohexane for 10 minutes to dissolve and remove hot-melt adhesive, and the non-woven fabric and the thermoplastic polyurethane elastic fiber are removed and placed on filter paper so as to be both tension-free, and dried for 12 hours in an environment of 20 °C and 65% RH. The total weight Y (g) of the non-woven fabric and the thermoplastic polyurethane elastic fiber after drying and the length Z (m) of the non-woven fabric are measured. The length Z (m) of the non-woven fabric refers to the length in the direction parallel to the direction of insertion of the thermoplastic polyurethane elastic fiber. From the following calculation formula, the amount (g/m) of hot-melt adhesive applied to the thermoplastic polyurethane elastic fibers is calculated:

$$\text{Amount of hot-melt adhesive (g/m)} = (X - Y) / Z$$

Measurement is performed four times and the average value thereof is taken.

(3) Full Elongation Rate of Gather Member

Using the length Z (m) of the non-woven fabric obtained in (2) above, the full elongation rate (%) of the gather member is calculated from the following calculation formula.

$$\text{Full elongation rate (\%)} = (10 \times Z - 1) \times 100$$

Measurement is performed four times and the average value thereof is taken.

(4) Total Fineness of Thermoplastic Polyurethane Elastic Fibers Contained in Gather Member

The length of the thermoplastic polyurethane elastic fiber removed from the gather member by the method described in (2) above in a relaxed state (hereinafter also referred to as "relaxation length") and the sample weight (g) are measured. The total fineness (dtex) of thermoplastic polyurethane elastic fibers in a relaxed state is calculated from the following calculation formula. Measurement is performed four times and the average value thereof is taken. Note that the "relaxed state" refers to a state in which the thermoplastic polyurethane elastic fiber removed from the gather member is allowed to stand in an unloaded state for 2 hours or longer.

$$\text{Total fineness A (dtex)} = \text{sample weight (g)} \times 10000 / \text{relaxation length (m)}$$

(5) Filament Number of Thermoplastic Polyurethane Elastic Fibers Contained in Gather Member>

The cross-section of the thermoplastic polyurethane elastic fiber removed from the gather member by the method described in (2) above is observed using an electron microscope JSM-6510 manufactured by JEOL Ltd., and the number of single strands is measured as the filament number.

(6) Single-Strand Fineness of Thermoplastic Polyurethane Elastic Fibers Contained in Gather Member

Using the total fineness A (dtex) and filament number obtained in (4) and (5) above, the single-strand fineness (dtex) of the thermoplastic polyurethane elastic fibers is calculated from the following calculation formula:

$$\text{Single-strand fineness (dtex)} = A / \text{filament number}$$

(7) Full Width at Half Maximum of Peak of Hard Domain Distance and Azimuth Distribution of Thermoplastic Polyurethane Elastic Fiber

The sample is set in a sample stand so that the fiber axis of the thermoplastic polyurethane elastic fiber removed from the gather member by the method described in (2) above is oriented horizontally. The fiber is irradiated with X-rays perpendicularly, and measurement is performed using an SAXS under the following conditions.

> Measuring device: NANOPIX manufactured by Rigaku Corporation
> Incident X-ray wavelength $\lambda$: 0.154 nm
> Detector: two-dimensional detector "Hypix-6000"
> Measurement time: 15 minutes
> Camera length: 1312 mm
> Optical system:
> Point collimation:
>
> first slit: 0.55 mm$\varphi$, guard slit: 0.35 mm$\varphi$
> High Resolution Mode
> Beam stopper: 2 mm$\varphi$

Setting the 12 o'clock direction of the two-dimensional SAXS pattern I ($2\theta$, $\varphi$) obtained by the two-dimensional detector as 0°, and defining the azimuth angle $\theta$ clockwise, the fan-shaped average scattering intensity I is calculated from the following formula:

[Math 1]

$$I(\,2\theta\,) = \frac{1}{2(\varphi_e - \varphi_s)}\left[\int_{\varphi s}^{\varphi e}\frac{I(2\theta,\varphi)}{cos^3\,2\,\theta}d\varphi + \int_{\varphi s+180}^{\varphi e+180}\frac{I(2\theta,\varphi)}{cos^3\,2\,\theta}d\varphi\right]$$

where $\varphi_s$: -10°, $\varphi_e$: 10°, and $\theta$: the Bragg angle.
The obtained fan-shaped average scattering intensity I is subjected to empty cell scattering correction using the following formula to calculate the one-dimensional scattering intensity $I_c$.

[Math 2]

$$I_c(2\theta) = C\left[\frac{I_{sample}(2\theta)}{t_{sample}T} - \frac{I_{empty}(2\theta)}{t_{empty}}\right]$$

where $I_c$ ($2\theta$): empty cell corrected scattering intensity, $I_{sample}$ ($2\theta$): scattering intensity of sample, $I_{empty}$ ($2\theta$): scattering intensity of empty cell, $t_{sample}$: sample measurement time, $t_{empty}$: measurement time of empty cell measurement, T: transmittance, and C: device constant.
Next, the distance between hard domains is calculated from the peak position $q_{max}$ obtained by fitting the vicinity of the peak of the one-dimensional scattering intensity $I_c$ with a Gaussian function. Note that q is the absolute value of the scattering vector.

[Math 3]

$$q = \frac{4\pi\sin\theta}{\lambda}$$

[Math 4]

$$\text{Distance between hard domains} = \frac{2\pi}{q\,max}$$

where $\theta$: the Bragg angle, and $\lambda$: the incident X-ray wavelength.
Next, the azimuthal distribution of the scattered light intensity when $q_s < q < q_e$ ($q_s = q_{max}$ - 0.1 nm$^{-1}$, $q_e = q_{max}$ + 0.1 nm$^{-1}$) is calculated from by the following formula:

[Math 5]

$$I(\varphi) = \int_{qs}^{qe} \left[ \frac{I_{sample}(q,\varphi)}{t_{sample}T} - \frac{I_{empty}(q,\varphi)}{t_{empty}} \right] dq$$

where $I_{sample}$ (q, $\varphi$): two-dimensional scattering pattern of the sample, and $I_{empty}$ (q, $\varphi$): two-dimensional scattering pattern of an empty cell.

The obtained azimuth angle distribution I ($\varphi$) is normalized from the following formula:

[Math 6]

$$I_N(\varphi) = \frac{I(\varphi)}{\int_{0°}^{360°} \frac{I(\varphi)}{360°} d\varphi}$$

In the normalized azimuth angle distribution $I_N(\varphi)$, the full width at half maximum is calculated from the peak observed closest to the azimuth angle $\varphi = 90°$ under the following conditions.

Analysis software: Igor Pro 8.00 manufactured by Wavemetrics
Fitting method: Igor Pro 8.00 Multi-peak Fit

The baseline is a constant, and the initial value is a constant connecting both ends of the tail of the peak. Furthermore, the baseline constant is used as a variable in the fitting. A Gaussian function is used as a fitting function, and the location, width, and height of the peak in Multi-peak Fit are used as variables, and the initial values are set to 90°, 50°, and 1, respectively. The fitting range is set at both ends of the tail of the peak observed closest to the azimuth angle $\varphi$. = 90°.

(8) Qualitative Determination of Constituent Components of Thermoplastic Polyurethane Contained in Gather Member and Measurement of Molecular Weight of Hard Segments

A sample removed from the thermoplastic polyurethane elastic fiber removed from the gather member by the method described in (2) above and a predetermined amount of internal standard dimethyl sulfoxide are measured by NMR under the following conditions, and the structures and molar ratios of the polymer polyol, chain extender, and diisocyanate are identified. The structures of the diisocyanate and chain extender can be determined from the peak positions determined by NMR measurement.

Measuring device: Bruker Biospin Avance 600
Measurement nucleus: 1H
Resonance frequency: 600 MHz
Accumulated number of repetitions: 256
Measurement temperature: room temperature
Solvent: Deuterated dimethylformamide
Measured concentration: 1.5% by weight
Chemical shift standard: dimethylformamide (8.0233 ppm)

The molecular weight of the hard segments of the thermoplastic polyurethane elastic fiber contained in the gather member is calculated by solving the following simultaneous formulas (1) and (2).

$$Mh = \{Mda(N1-1) + Mdi \times N0\} / (N1 - N0 - 1) + 2Mdi \qquad ...\text{formula (1)}$$

$$N0 = 0.03806N1^4 - 0.3997N1^3 + 1.617N1^2 - 2.144N1^1 + 0.8795 \quad ...\text{formula (2)}$$

Mda: Molecular weight of chain extender (when using a mixture of two or more types, the number average molecular weight)

Mdi: molecular weight of isocyanate
N0: molar ratio of unreacted isocyanate to polymer polyol
N1: molar ratio of isocyanate to polymer polyol
Mh: Molecular weight of hard segments

(9) Determination of Presence or Absence of Crosslinks Containing Allophanate Bond in Thermoplastic Polyurethane Elastic Fibers Contained in Gather Member

A thermoplastic polyurethane elastic fiber which dissolves in the DMAc dissolution test described below and which is not confirmed to have an allophanate bond in the NMR measurement described below is determined to "have no crosslinks containing an allophanate bond" as used herein. A thermoplastic polyurethane elastic fiber which does not dissolve in the DMAc dissolution test below or which dissolves in the DMAc dissolution test below but is confirmed to have an allophanate bond in the NMR measurement below, is determined to "have a crosslink containing an allophanate bond.

<DMAc Dissolution Test>

0.2 g of a thermoplastic polyurethane elastic fiber removed from a gather member by the method described in (2) above is accurately weighed, immersed in 10 g of DMAc, and stirred at 20 °C for 48 hours. After stirring, if no clump-like polymer having a diameter of 1 mm or more can be visually confirmed, it is determined that the polymer is dissolved in the DMAc.

<NMR Measurement (Qualification of Allophanate Bond)>

Predetermined amounts of a polyurethane elastic fiber removed from a gather member by the method described in (2) above and dried at 80 °C for 5 hours under a vacuum of -0.1 MPa and dimethyl sulfoxide as an internal standard are weighed out and measured by NMR under the following conditions. By this measurement, the ratio of allophanate bonds to urethane bonds is confirmed. The ratio of allophanate bonds to the urethane bonds can be calculated by comparing the respective hydrogen integral values, and when this ratio is less than 0.05%, it is defined that no allophanate groups are contained. Generally, hydrogen signals of allophanate bonds are often observed at 10.5 to 11.0 ppm, but this is not always the case.

[NMR Measurement Conditions]

Measuring device: ECS400 manufactured by JEOL
Nucleus: 1H
Resonance frequency: 400 MHz
Accumulated number of repetitions: 256
Measurement temperature: room temperature
Solvent: Deuterated dimethylformamide
Measured concentration: 1.5% by weight
Chemical shift standard: dimethylformamide (8.0233 ppm)

(10) Qualification of Chemical Structure of Hot-Melt Adhesive Contained in Gather Member

The hot-melt adhesive solution eluted from the gather member by the method described in (2) above is concentrated and dried using an evaporator to prepare a sample. A predetermined amount of the sample is weighed and measured by NMR under the following conditions to identify the chemical structure.

Measuring device: Bruker Biospin Avance 600
Measurement nucleus: 1H
Resonance frequency: 600 MHz
Accumulated number of repetitions: 256
Measurement temperature: room temperature
Solvent: Deuterated chloroform
Measured concentration: 1.5% by weight

Chemical shift standard: Chloroform (7.24 ppm)

(11) Basis Weight of Non-Woven Fabric Contained in Gather Member

Non-woven fabric removed from a gather member by the method described in (2) above is cut to 0.5 cm × 10 cm, the weight (g) of the non-woven fabric is measured, and the basis weight (g/m$^2$) of the non-woven fabric contained in the gather member is calculated from the following calculation formula:

$$\text{Basis weight of non-woven fabric (g/m}^2) = \text{weight of non-woven fabric (g)} / 0.0005 \text{ (m}^2)$$

The measurement is performed 10 times and the average value thereof is taken.

(12) Unlikeliness of Shift of Gather Member During Movement

A gather member produced as described above is allowed to stand in an unelongated state for one day to sufficiently relax the stress in the elastic fibers. Thereafter, a ring having a length of 50 cm is produced from the relaxed gather member. This ring is worn on the waist by 10 subjects having waist lengths of 75 cm to 80 cm, the number of subjects who feel that the ring is unlikely to shift when worn for 8 hours is measured in a sensory evaluation. In the above sensory evaluation, the greater the number of subjects who feel that the ring is unlikely to shift, the more unlikely the gather member is to shift. In the present Examples, a gather member for which the number of subjects who feel that the ring is unlikely to shift is 5 or more is evaluated as a pass.

(13) Unlikeliness of Marks Left When Gather Member is Worn

A gather member produced as described above is allowed to stand in an unelongated state for one day to sufficiently relax the stress in the elastic fibers. Thereafter, a ring having a length of 50 cm is produced from the relaxed gather member. This ring is worn on the waist by 10 subjects having waist lengths of 75 cm to 80 cm, and the number of subjects who have no wear marks after wearing for 8 hours is measured. In the above sensory evaluation, the greater the number of subjects who have no marks, the more unlikely the gather member is to leave marks. In the present Examples, a gather member for which the number of subjects who have no wear marks is 5 or more is evaluated as a pass.

[Example 1]

2400 g of polytetramethylene ether diol having a number average molecular weight of 1800 and 775.78 g of 4,4'-diphenylmethane diisocyanate were reacted by stirring at 60 °C for 3 hours in a dry nitrogen atmosphere to obtain a polyurethane prepolymer capped with terminal isocyanate. 162.01 g of 1,4-butanediol was added to this polyurethane prepolymer and stirred for 15 minutes to obtain a thermoplastic polyurethane having a viscosity of 2000 poise (30 °C).
Thereafter, the thermoplastic polyurethane was dispensed onto a Teflon™ tray and annealed in a hot air oven at 110 °C for 19 hours while remaining in the tray to obtain a thermoplastic polyurethane resin. This thermoplastic polyurethane resin had a weight average molecular weight of 200,000 in GPC.
The thermoplastic polyurethane resin obtained in this manner was pulverized into a powder of approximately 3 mm using a UG-280 pulverizer manufactured by Horai Co., Ltd. After the pulverized chips were dried in a dehumidifying dryer at a temperature of 110 °C to a moisture content of 100 ppm, thermoplastic polyurethane resin powder was introduced from a hopper and melted in an extruder. The mixture was measured and pressurized using a gear pump installed in the head, filtered through a filter, and then discharged from a nozzle having a diameter of 0.23 mm and 60 holes at a rate of 31 g/min. Thereafter, cold air was blown from a cold air chamber in which the length and speed of the cold air were adjusted, and applied perpendicularly to the fibers during melt-spinning. Using a ring-type false twisting machine, the multifilaments were twisted and wound onto a paper tube while being treated with a treatment agent mainly composed of polydimethylsiloxane and mineral oil to obtain a wound body of thermoplastic polyurethane elastic fibers of 620 dtex/60 filaments.
Five thermoplastic polyurethane elastic fibers were arranged in parallel with an interval of 7 mm and elongated to a length 2.0 times the original length, the polyurethane elastic fibers were continuously coated with a hot-melt adhesive (765E manufactured by Henkel Japan Co., Ltd.) melted at 150 °C using a V-slit, the polyurethane elastic fibers coated with the hot-melt adhesive were continuously interposed between two non-woven fabrics (Eltas Guard™ manufactured by Asahi Kasei Corporation) having a width of 30 cm and a basis weight of 17 g/m$^2$, continuously press-bonded from above with a pair of heating rollers having an outer diameter of 16 cm and a width of 40 cm and consisting of one roller having a surface temperature set to 80 °C and the other roller having a surface of room temperature by an air cylinder (CQ2WB 100-50DZ manufactured by SMC Corporation) supplied with an air pressure of 0.5 MPa, and cold air was blown at a speed of 0.6 m/s and a temperature of 16 °C from a cold air chamber having a length of 300 mm at a distance of 300 to 600 mm arranged below the press-bonding so as to apply the cold air perpendicularly to the gather member, whereby a gather member was produced.

The speed response ratio of the obtained gather member was 1.50, and the full elongation rate was 100%. The thermoplastic polyurethane elastic fibers contained in the gather member had 620 dtex and 60 filaments and contained polyurethane synthesized from polytetramethylene ether glycol (PTMG), 4,4'-diphenylmethane diisocyanate (MDI), and 1,4-butanediol (BDO), the distance between hard domains was 12.0 nm, the full width at half maximum of the peak of the azimuthal angle distribution was 90°, the molecular weight of the hard segments was 1005, and no crosslinked structure was contained. The hot-melt adhesive was a styrene-butadiene-styrene block copolymer (SBS), the coating amount thereof was 0.04 g/m$^2$, and the basis weight of the non-woven fabric was 17 g/m$^2$. The results are also shown in Table 1 below.

[Examples 2 to 9]

Gather members were produced in the same manner as in Example 1, except that in a production step of the gather member, the speed response ratio of the gather member was adjusted by changing the temperature and air pressure of the heated rollers for press-bonding to control the distance between the hard domains of the thermoplastic polyurethane elastic fibers.

[Examples 10 to 16]

Gather members were produced in the same manner as in Example 1, except that in a production step of the gather member, the speed response ratio of the gather member was adjusted by changing the cold air speed and temperature of the cold air chamber to control the full width at half maximum of the peak of the azimuthal angle distribution of thermoplastic polyurethane elastic fibers.

[Examples 17 to 27]

Gather members were produced in the same manner as in Example 1, except for using thermoplastic polyurethane elastic fibers different in filament number, single-strand fineness, or total fineness.

[Examples 28 and 29]

Gather members were produced in the same manner as in Example 1, except that 1,6-hexamethylene diisocyanate (HDI) was used as the constituent isocyanate (Example 28) or 1,3-propanediol (PDO) was used as the chain extender (Example 29) for thermoplastic polyurethane elastic fibers used.

[Examples 30 to 36]

Gather members were produced in the same manner as in Example 1, except for using thermoplastic polyurethane elastic fibers different in the molecular weight of hard segments.

[Example 37]

A gather member was produced in the same manner as in Example 1, except for using thermoplastic polyurethane elastic fibers having a crosslinked structure.

[Examples 38 to 42]

Gather members were produced in the same manner as in Example 1, except that the amount of hot-melt adhesive applied was changed.

[Examples 43 to 47]

Gather members were produced in the same manner as in Example 1, except that, as the hot-melt adhesive, a styrene-isoprene-styrene block copolymer (SIS) (Example 43), styrene-butadiene/butylene-styrene block copolymer (SBBS) (Example 44), styrene-ethylene/butylene-styrene block copolymer (SEBS) (Example 45), ethylene-ethyl acrylate copolymer (EEA) (Example 46), or ethylene-vinyl acetate copolymer (EVA) (Example 47) was used.

[Examples 48 to 51]

Gather members were produced in the same manner as in Example 1, except for using non-woven fabrics having different in basis weight.

[Examples 52 to 55]

Gather members were produced in the same manner as in Example 1, except that the draw ratios of the thermoplastic polyurethane elastic fibers before bonding to the non-woven fabric were changed to change the full elongation rates.

[Comparative Examples 1 and 2]

Gather members were produced in the same manner as in Example 1, except that in a production step of the gather member, the speed response ratio of the gather member was adjusted by changing the temperature and air pressure of the heated rollers for press-bonding to control the distance between the hard domains of the thermoplastic polyurethane elastic fibers.

[Comparative Examples 3 and 4]

Gather members were produced in the same manner as in Example 1, except that in a production step of the gather member, the speed response ratio of the gather member was adjusted by changing the cold air speed and temperature of the cold air chamber to control the full width at half maximum of the peak of the azimuthal angle distribution of thermoplastic polyurethane elastic fibers.

[Comparative Example 5]

A gather member was produced in the same manner as in Example 1, except that polyurethane elastic fibers produced by dry spinning in which the chain extender composed of an active hydrogen compound was ethylenediamine (EDA) and the molecular weight of the hard segments was 700 were used. The distance between hard domains of polyurethane elastic fibers pulled out from the gather member was 9.0 nm and the speed response ratio was 1.00.

[Comparative Example 6]

A gather member was produced in the same manner as in Example 1, except that in a production step of the gather member, the temperature of the heating rollers for press-bonding was set to room temperature and the cold air chamber was not used.

[Comparative Example 7]

A gather member was produced in the same manner as in Comparative Example 6, except for using polyurethane elastic fibers produced by dry spinning, in which the chain extender composed of an active hydrogen compound was ethylenediamine (EDA) and the molecular weight of the hard segments was 700.
The production conditions and the measurement results of the properties of the obtained polyurethane elastic fibers and gather members of each of the Examples and Comparative Examples described above are shown in Tables 1 to 5 below.

Table 1]

| | | Ex 1 | Ex 2 | Ex 3 | Ex 4 | Ex 5 | Ex 6 | Ex 7 | Ex 8 | Ex 9 | Ex 10 | Ex 11 | Ex 12 | Ex 13 | Ex 14 | Ex 15 | Ex 16 |
|---|---|---|---|---|---|---|---|---|---|---|---|---|---|---|---|---|---|
| Gather member | Speed response ratio | 1.50 | 1.05 | 1.10 | 1.20 | 2.00 | 2.20 | 2.80 | 2.90 | 3.00 | 1.06 | 1.10 | 1.20 | 2.00 | 2.50 | 2.50 | 3.00 |
| | Full elongation rate (%) | 100 | 100 | 100 | 100 | 100 | 100 | 100 | 100 | 100 | 100 | 100 | 100 | 100 | 100 | 100 | 100 |

(continued)

| | | Ex 1 | Ex 2 | Ex 3 | Ex 4 | Ex 5 | Ex 6 | Ex 7 | Ex 8 | Ex 9 | Ex 10 | Ex 11 | Ex 12 | Ex 13 | Ex 14 | Ex 15 | Ex 16 |
|---|---|---|---|---|---|---|---|---|---|---|---|---|---|---|---|---|---|
| Thermoplastic polyurethane elastic fibers | Distance between hard domains (nm) | 12.0 | 10.0 | 10.5 | 11.0 | 15.0 | 20.0 | 25.0 | 27.0 | 30.0 | 12.0 | 12.0 | 12.0 | 12.0 | 12.0 | 12.0 | 12.0 |
| | Full width at half maximum of peak of azimuthal angle distribution (°) | 90 | 90 | 90 | 90 | 90 | 90 | 90 | 90 | 90 | 50 | 60 | 80 | 100 | 110 | 120 | 130 |
| | Filament number | 60 | 60 | 60 | 60 | 60 | 60 | 60 | 60 | 60 | 60 | 60 | 60 | 60 | 60 | 60 | 60 |
| | Single-strand fineness (dtex) | 10.3 | 10.3 | 10.3 | 10.3 | 10.3 | 10.3 | 10.3 | 10.3 | 10.3 | 10.3 | 10.3 | 10.3 | 10.3 | 10.3 | 10.3 | 10.3 |
| | Total fineness (dtex) | 620 | 620 | 620 | 620 | 620 | 620 | 620 | 620 | 620 | 620 | 620 | 620 | 620 | 620 | 620 | 620 |
| | Polymer polyol used for synthesis | PTMG | PTMG | PTMG | PTMG | PTMG | PTMG | PTMG | PTMG | PTMG | PTMG | PTMG | PTMG | PTMG | PTMG | PTMG | PTMG |
| | Diisocyanate used for synthesis | MDI | MDI | MDI | MDI | MDI | MDI | MDI | MDI | MDI | MDI | MDI | MDI | MDI | MDI | MDI | MDI |
| | Chain extender used for synthesis | BDO | BDO | BDO | BDO | BDO | BDO | BDO | BDO | BDO | BDO | BDO | BDO | BDO | BDO | BDO | BDO |
| | Molecular weight of hard segments | 1005 | 1005 | 1005 | 1005 | 1005 | 1005 | 1005 | 1005 | 1005 | 1005 | 1005 | 1005 | 1005 | 1005 | 1005 | 1005 |
| | Presence or absence of crosslinked structure | Absent | Absent | Absent | Absent | Absent | Absent | Absent | Absent | Absent | Absent | Absent | Absent | Absent | Absent | Absent | Absent |

| | | Ex 1 | Ex 2 | Ex 3 | Ex 4 | Ex 5 | Ex 6 | Ex 7 | Ex 8 | Ex 9 | Ex 10 | Ex 11 | Ex 12 | Ex 13 | Ex 14 | Ex 15 | Ex 16 |
|---|---|---|---|---|---|---|---|---|---|---|---|---|---|---|---|---|---|
| Hot-melt adhesive | Content (g/m) | 0.04 | 0.04 | 0.04 | 0.04 | 0.04 | 0.04 | 0.04 | 0.04 | 0.04 | 0.04 | 0.04 | 0.04 | 0.04 | 0.04 | 0.04 | 0.04 |
| | Chemical structure | SBS | SBS | SBS | SBS | SBS | SBS | SBS | SBS | SBS | SBS | SBS | SBS | SBS | SBS | SBS | SBS |
| Non-woven fabric | Basis weight (g/m$^2$) | 17 | 17 | 17 | 17 | 17 | 17 | 17 | 17 | 17 | 17 | 17 | 17 | 17 | 17 | 17 | 17 |
| Usability evaluation | Unlikeliness of shift of gather member during movement | 10 | 5 | 8 | 10 | 10 | 10 | 10 | 10 | 10 | 6 | 8 | 10 | 10 | 10 | 10 | 10 |
| | Unlikeliness of marks left when gather member is worn | 10 | 10 | 10 | 10 | 10 | 9 | 7 | 6 | 5 | 10 | 10 | 10 | 10 | 9 | 8 | 5 |

[Table 2]

| | | Ex 17 | Ex 18 | Ex 19 | Ex 20 | Ex 21 | Ex 22 | Ex 23 | Ex 24 | Ex 25 | Ex 26 | Ex 27 | Ex 28 | Ex 29 |
|---|---|---|---|---|---|---|---|---|---|---|---|---|---|---|
| Gather member | Speed response ratio | 1.05 | 1.15 | 1.20 | 1.20 | 1.07 | 1.05 | 1.05 | 1.50 | 1.50 | 1.05 | 1.05 | 1.06 | 1.06 |
| | Full elongation rate (%) | 100 | 100 | 100 | 100 | 100 | 100 | 100 | 100 | 100 | 100 | 100 | 100 | 100 |

| | | Ex 17 | Ex 18 | Ex 19 | Ex 20 | Ex 21 | Ex 22 | Ex 23 | Ex 24 | Ex 25 | Ex 26 | Ex 27 | Ex 28 | Ex 29 |
|---|---|---|---|---|---|---|---|---|---|---|---|---|---|---|
| Thermoplastic polyurethane elastic fibers | Distance between hard domains (nm) | 12.0 | 12.0 | 12.0 | 12.0 | 12.0 | 12.0 | 12.0 | 12.0 | 12.0 | 12.0 | 12.0 | 12.0 | 12.0 |
| | Full width at half maximum of peak of azimuthal angle distribution (°) | 90 | 90 | 90 | 90 | 90 | 90 | 90 | 90 | 90 | 90 | 90 | 90 | 90 |
| | Filament number | 30 | 32 | 40 | 80 | 120 | 128 | 12 | 15 | 150 | 180 | 20 | 60 | 60 |
| | Single-strand fineness (dtex) | 20.7 | 19.4 | 15.5 | 7.75 | 5.2 | 4.8 | 10.0 | 10.0 | 100 | 100 | 7.5 | 10.3 | 10.3 |
| | Total fineness (dtex) | 620 | 620 | 620 | 620 | 620 | 620 | 120 | 150 | 1500 | 1800 | 150 | 620 | 620 |
| | Polymer polyol used for synthesis | PTMG | PTMG | PTMG | PTMG | PTMG | PTMG | PTMG | PTMG | PTMG | PTMG | PTMG | PTMG | PTMG |
| | Diisocyanate used for synthesis | MDI | MDI | MDI | MDI | MDI | MDI | MDI | MDI | MDI | MDI | MDI | HDI | MDI |
| | Chain extender used for synthesis | BDO | BDO | BDO | BDO | BDO | BDO | BDO | BDO | BDO | BDO | BDO | BDO | PDO |
| | Molecular weight of hard segments | 1005 | 1005 | 1005 | 1005 | 1005 | 1005 | 1005 | 1005 | 1005 | 1005 | 1005 | 1005 | 1005 |
| | Presence or absence of crosslinked structure | Absent | Absent | Absent | Absent | Absent | Absent | Absent | Absent | Absent | Absent | Absent | Absent | Absent |

(continued)

| | | Ex 17 | Ex 18 | Ex 19 | Ex 20 | Ex 21 | Ex 22 | Ex 23 | Ex 24 | Ex 25 | Ex 26 | Ex 27 | Ex 28 | Ex 29 |
|---|---|---|---|---|---|---|---|---|---|---|---|---|---|---|
| Hot-melt adhesive | Content (g/m) | 0.04 | 0.04 | 0.04 | 0.04 | 0.04 | 0.04 | 0.04 | 0.04 | 0.04 | 0.04 | 0.04 | 0.04 | 0.04 |
| | Chemical structure | SBS | SBS | SBS | SBS | SBS | SBS | SBS | SBS | SBS | SBS | SBS | SBS | SBS |
| Non-woven fabric | Basis weight $(g/m^2)$ | 17 | 17 | 17 | 17 | 17 | 17 | 17 | 17 | 17 | 17 | 17 | 17 | 17 |
| Usability evaluation | Unlikeliness of shift of gather member during movement | 5 | 9 | 10 | 10 | 7 | 5 | 5 | 10 | 10 | 5 | 5 | 6 | 6 |
| | Unlikeliness of marks left when gather member is worn | 10 | 10 | 10 | 10 | 10 | 10 | 10 | 10 | 10 | 10 | 10 | 10 | 10 |

[Table 3]

| Gather member | | Ex 30 | Ex 31 | Ex 32 | Ex 33 | Ex 34 | Ex 35 | Ex 36 | Ex 37 | Ex 38 | Ex 39 | Ex 40 | Ex 41 | Ex 42 |
|---|---|---|---|---|---|---|---|---|---|---|---|---|---|---|
| | Speed response ratio | 1.06 | 1.07 | 1.20 | 2.00 | 2.50 | 2.80 | 3.00 | 1.06 | 1.07 | 1.10 | 1.20 | 1.20 | 1.07 |
| | Full elongation rate (%) | 100 | 100 | 100 | 100 | 100 | 100 | 100 | 100 | 100 | 100 | 100 | 100 | 100 |

| | | Ex 30 | Ex 31 | Ex 32 | Ex 33 | Ex 34 | Ex 35 | Ex 36 | Ex 37 | Ex 38 | Ex 39 | Ex 40 | Ex 41 | Ex 42 |
|---|---|---|---|---|---|---|---|---|---|---|---|---|---|---|
| Thermoplastic polyurethane elastic fibers | Distance between hard domains (nm) | 12.0 | 12.0 | 12.0 | 12.0 | 12.0 | 12.0 | 12.0 | 12.0 | 12.0 | 12.0 | 12.0 | 12.0 | 12.0 |
| | Full width at half maximum of peak of azimuthal angle distribution (°) | 90 | 90 | 90 | 90 | 90 | 90 | 90 | 90 | 90 | 90 | 90 | 90 | 90 |
| | Filament number | 60 | 60 | 60 | 60 | 60 | 60 | 60 | 60 | 60 | 60 | 60 | 60 | 60 |
| | Single-strand fineness (dtex) | 10.3 | 10.3 | 10.3 | 10.3 | 10.3 | 10.3 | 10.3 | 10.3 | 10.3 | 10.3 | 10.3 | 10.3 | 10.3 |
| | Total fineness (dtex) | 620 | 620 | 620 | 620 | 620 | 620 | 620 | 620 | 620 | 620 | 620 | 620 | 620 |
| | Polymer polyol used for synthesis | PTMG | PTMG | PTMG | PTMG | PTMG | PTMG | PTMG | PTMG | PTMG | PTMG | PTMG | PTMG | PTMG |
| | Diisocyanate used for synthesis | MDI | MDI | MDI | MDI | MDI | MDI | MDI | MDI | MDI | MDI | MDI | MDI | MDI |
| | Chain extender used for synthesis | BDO | BDO | BDO | BDO | BDO | BDO | BDO | BDO | BDO | BDO | BDO | BDO | BDO |
| | Molecular weight of hard segments | 730 | 750 | 850 | 1200 | 1300 | 1500 | 1550 | 1005 | 1005 | 1005 | 1005 | 1005 | 1005 |
| | Presence or absence of crosslinked structure | Absent | Absent | Absent | Absent | Absent | Absent | Absent | Present | Absent | Absent | Absent | Absent | Absent |

| | | Ex 30 | Ex 31 | Ex 32 | Ex 33 | Ex 34 | Ex 35 | Ex 36 | Ex 37 | Ex 38 | Ex 39 | Ex 40 | Ex 41 | Ex 42 |
|---|---|---|---|---|---|---|---|---|---|---|---|---|---|---|
| Hot-melt adhesive | Content (g/m) | 0.04 | 0.04 | 0.04 | 0.04 | 0.04 | 0.04 | 0.04 | 0.04 | 0.01 | 0.02 | 0.03 | 0.08 | 0.12 |
| | Chemical structure | SBS | SBS | SBS | SBS | SBS | SBS | SBS | SBS | SBS | SBS | SBS | SBS | SBS |
| Non-woven fabric | Basis weight (g/m$^2$) | 17 | 17 | 17 | 17 | 17 | 17 | 17 | 17 | 17 | 17 | 17 | 17 | 17 |
| Usability evaluation | Unlikeliness of shift of gather member during movement | 6 | 7 | 10 | 10 | 10 | 10 | 10 | 6 | 7 | 8 | 10 | 10 | 7 |
| | Unlikeliness of marks left when gather member is worn | 10 | 10 | 10 | 10 | 8 | 7 | 6 | 10 | 10 | 10 | 10 | 10 | 10 |

EP 4 458 336 A1

[Table 4]

| | | Ex 43 | Ex 44 | Ex 45 | Ex 46 | Ex 47 | Ex 48 | Ex 49 | Ex 50 | Ex 51 | Ex 52 | Ex 53 | Ex 54 | Ex 55 |
|---|---|---|---|---|---|---|---|---|---|---|---|---|---|---|
| Gather member | Speed response ratio | 1.50 | 1.50 | 1.50 | 1.10 | 1.10 | 1.15 | 1.50 | 1.50 | 1.15 | 1.50 | 1.50 | 1.50 | 1.15 |
| | Full elongation rate (%) | 100 | 100 | 100 | 100 | 100 | 100 | 100 | 100 | 100 | 50 | 200 | 250 | 270 |

|  |  | Ex 43 | Ex 44 | Ex 45 | Ex 46 | Ex 47 | Ex 48 | Ex 49 | Ex 50 | Ex 51 | Ex 52 | Ex 53 | Ex 54 | Ex 55 |
|---|---|---|---|---|---|---|---|---|---|---|---|---|---|---|
| Thermoplastic polyurethane elastic fibers | Distance between hard domains (nm) | 12.0 | 12.0 | 12.0 | 12.0 | 12.0 | 12.0 | 12.0 | 12.0 | 12.0 | 12.0 | 12.0 | 12.0 | 12.0 |
|  | Full width at half maximum of peak of azimuthal angle distribution (°) | 90 | 90 | 90 | 90 | 90 | 90 | 90 | 90 | 90 | 90 | 90 | 90 | 90 |
|  | Filament number | 60 | 60 | 60 | 60 | 60 | 60 | 60 | 60 | 60 | 60 | 60 | 60 | 60 |
|  | Single-strand fineness (dtex) | 10.3 | 10.3 | 10.3 | 10.3 | 10.3 | 10.3 | 10.3 | 10.3 | 10.3 | 10.3 | 10.3 | 10.3 | 10.3 |
|  | Total fineness (dtex) | 620 | 620 | 620 | 620 | 620 | 620 | 620 | 620 | 620 | 620 | 620 | 620 | 620 |
|  | Polymer polyol used for synthesis | PTMG | PTMG | PTMG | PTMG | PTMG | PTMG | PTMG | PTMG | PTMG | PTMG | PTMG | PTMG | PTMG |
|  | Diisocyanate used for synthesis | MDI | MDI | MDI | MDI | MDI | MDI | MDI | MDI | MDI | MDI | MDI | MDI | MDI |
|  | Chain extender used for synthesis | BDO | BDO | BDO | BDO | BDO | BDO | BDO | BDO | BDO | BDO | BDO | BDO | BDO |
|  | Molecular weight of hard segments | 1005 | 1005 | 1005 | 1005 | 1005 | 1005 | 1005 | 1005 | 1005 | 1005 | 1005 | 1005 | 1005 |
|  | Presence or absence of crosslinked structure | Absent | Absent | Absent | Absent | Absent | Absent | Absent | Absent | Absent | Absent | Absent | Absent | Absent |

EP 4 458 336 A1

(continued)

| | | Ex 43 | Ex 44 | Ex 45 | Ex 46 | Ex 47 | Ex 48 | Ex 49 | Ex 50 | Ex 51 | Ex 52 | Ex 53 | Ex 54 | Ex 55 |
|---|---|---|---|---|---|---|---|---|---|---|---|---|---|---|
| Hot-melt adhesive | Content (g/m) | 0.04 | 0.04 | 0.04 | 0.04 | 0.04 | 0.04 | 0.04 | 0.04 | 0.04 | 0.04 | 0.04 | 0.04 | 0.04 |
| | Chemical structure | SIS | SBBS | SEBS | EEA | EVA | SBS | SBS | SBS | SBS | SBS | SBS | SBS | SBS |
| Non-woven fabric | Basis weight $(g/m^2)$ | 17 | 17 | 17 | 17 | 17 | 7 | 8 | 50 | 60 | 17 | 17 | 17 | 17 |
| Usability evaluation | Unlikeliness of shift of gather member during movement | 10 | 10 | 10 | 8 | 8 | 9 | 10 | 10 | 9 | 10 | 10 | 10 | 9 |
| | Unlikeliness of marks left when gather member is worn | 10 | 10 | 10 | 10 | 10 | 10 | 10 | 10 | 10 | 10 | 10 | 10 | 10 |

[Table 5]

| | | Comp Ex 1 | Comp Ex 2 | Comp Ex 3 | Comp Ex 4 | Comp Ex 5 | Comp Ex 6 | Comp Ex 7 |
|---|---|---|---|---|---|---|---|---|
| Gather member | Speed response ratio | 1.00 | 3.20 | 1.00 | 1.00 | 1.00 | 1.00 | 1.00 |
| | Full elongation rate (%) | 100 | 100 | 100 | 100 | 100 | 100 | 100 |
| Thermoplastic polyurethane elastic fibers | Distance between hard domains (nm) | 9.0 | 35.0 | 12.0 | 12.0 | 9.0 | 9.5 | 9.0 |
| | Full width at half maximum of peak of azimuthal angle distribution (°) | 90 | 90 | 40 | 140 | 90 | 40 | 90 |
| | Filament number | 60 | 60 | 60 | 60 | 60 | 60 | 60 |
| | Single-strand fineness (dtex) | 10.3 | 10.3 | 10.3 | 10.3 | 10.3 | 10.3 | 10.3 |
| | Total fineness (dtex) | 620 | 620 | 620 | 620 | 620 | 620 | 620 |
| | Polymer polyol used for synthesis | PTMG | PTMG | PTMG | PTMG | PTMG | PTMG | PTMG |
| | Diisocyanate used for synthesis | MDI | MDI | MDI | MDI | MDI | MDI | MDI |
| | Chain extender used for synthesis | BDO | BDO | BDO | BDO | EDA | BDO | EDA |
| | Molecular weight of hard segments | 1005 | 1005 | 1005 | 1005 | 700 | 1005 | 700 |
| | Presence or absence of crosslinked structure | Absent | Absent | Absent | Absent | Absent | Absent | Absent |
| Hot-melt adhesive | Content (g/m) | 0.04 | 0.04 | 0.04 | 0.04 | 0.04 | 0.04 | 0.04 |
| | Chemical structure | SBS | SBS | SBS | SBS | SBS | SBS | SBS |
| Non-woven fabric | Basis weight (g/m$^2$) | 17 | 17 | 17 | 17 | 17 | 17 | 17 |
| Usability evaluation | Unlikeliness of shift of gather member during movement | 4 | 10 | 4 | 4 | 4 | 4 | 4 |
| | Unlikeliness of marks left when gather member is worn | 10 | 4 | 10 | 10 | 10 | 10 | 10 |

INDUSTRIAL APPLICABILITY

The gather member according to the present invention can exhibit an appropriate speed response ratio when used as a member of hygiene products such as absorbent articles represented by sanitary products and disposable paper diapers, whereby a product which is unlikely to shift and unlikely to leave marks caused by the strenuous movements of the wearer can be obtained.

## Claims

1. A gather member, wherein a ratio of a 50% elongation stress at a 2000%/min elongation speed to a 50% elongation stress at a 200%/min elongation speed is 1.05 or more and 3.00 or less.

2. The gather member according to claim 1, comprising at least thermoplastic polyurethane elastic fibers and a non-woven fabric.

3. The gather member according to claim 2, further comprising a hot-melt adhesive.

4. The gather member according to claim 2 or 3, wherein a distance between hard domains of the thermoplastic polyurethane elastic fibers as measured with a small-angle X-ray scattering device is 10.0 nm or more and 30.0 nm or less, and a full width at half maximum of a peak of an azimuthal angle distribution is 50° or more and 130° or less.

5. The gather member according to claim 2 or 3, wherein a filament number of the thermoplastic polyurethane elastic fibers is 10 or more and 150 or less.

6. The gather member according to claim 2 or 3, wherein a single-strand fineness of the thermoplastic polyurethane elastic fibers is 5 dtex or more and 20 dtex or less.

7. The gather member according to claim 2 or 3, wherein a total fineness of the thermoplastic polyurethane elastic fibers is 150 dtex or more and 1500 dtex or less.

8. The gather member according to claim 2 or 3, wherein the thermoplastic polyurethane elastic fibers contain a polyurethane synthesized from polymer polyol, MDI, and 1,4-butanediol.

9. The gather member according to claim 2 or 3, wherein a molecular weight of hard segments of the thermoplastic polyurethane elastic fibers is 750 or more and 1500 or less.

10. The gather member according to claim 2 or 3, wherein the thermoplastic polyurethane elastic fibers have substantially no crosslinks containing an allophanate bond.

11. The gather member according to claim 3, wherein a content of the hot-melt adhesive is 0.02 g/m or more and 0.10 g/m or less relative to a length upon elongation per thermoplastic polyurethane elastic fiber.

12. The gather member according to claim 3, wherein the hot-melt adhesive is a styrene block copolymer selected from the group consisting of block copolymers of a vinyl aromatic hydrocarbon and a conjugated diene compound and hydrogenated products thereof.

13. The gather member according to claim 2 or 3, wherein a basis weight of the non-woven fabric is 8 g/m$^2$ or more and 50 g/m$^2$ or less.

14. The gather member according to any one of claims 1 to 3, wherein a full elongation rate of the gather member is 50% or more and 250% or less.

15. A hygiene product comprising the gather member according to any one of claims 1 to 3.

## INTERNATIONAL SEARCH REPORT

| International application No. |
|---|
| **PCT/JP2022/047747** |

**A. CLASSIFICATION OF SUBJECT MATTER**

*A61F 13/49*(2006.01)i; *D01F 6/70*(2006.01)i
FI: A61F13/49 312Z; D01F6/70 B; A61F13/49 311Z

According to International Patent Classification (IPC) or to both national classification and IPC

**B. FIELDS SEARCHED**

Minimum documentation searched (classification system followed by classification symbols)

A61F13/49; D01F6/70

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2017-205433 A (ASAHI KASEI CORP.) 24 November 2017 (2017-11-24) paragraphs [0018], [0022], [0024], [0042], [0049] | 1-3, 5-9, 11-15 |
| Y | | 4, 10 |
| Y | WO 2018/221490 A1 (BANDO CHEMICAL INDUSTRIES, LTD.) 06 December 2018 (2018-12-06) paragraph [0037] | 4 |
| A | | 1-3, 5-15 |
| Y | JP 2003-147666 A (TOYOBO CO., LTD.) 21 May 2003 (2003-05-21) paragraphs [0012], [0013] | 4 |
| A | | 1-3, 5-15 |
| Y | JP 2019-034070 A (LIVEDO CORP.) 07 March 2019 (2019-03-07) paragraphs [0033], [0034] | 10 |
| A | | 1-9, 11-15 |

☐ Further documents are listed in the continuation of Box C.  ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **27 February 2023** | **28 March 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

INTERNATIONAL SEARCH REPORT
Information on patent family members

International application No.

**PCT/JP2022/047747**

| Patent document cited in search report | | | Publication date (day/month/year) | Patent family member(s) | | | Publication date (day/month/year) |
|---|---|---|---|---|---|---|---|
| JP | 2017-205433 | A | 24 November 2017 | (Family: none) | | | |
| WO | 2018/221490 | A1 | 06 December 2018 | US | 2020/0339840 | A1 | |
| | | | | paragraph [0094] | | | |
| JP | 2003-147666 | A | 21 May 2003 | (Family: none) | | | |
| JP | 2019-034070 | A | 07 March 2019 | (Family: none) | | | |

Form PCT/ISA/210 (patent family annex) (January 2015)

**EP 4 458 336 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004081365 A